(19) Europäisches Patentamt
European Patent Office
Office européen des brevets

(11) Publication number: **0 154 298**
**A2**

# (12) EUROPEAN PATENT APPLICATION

(21) Application number: 85102240.0

(22) Date of filing: 28.02.85

(51) Int. Cl.[4]: A 61 K 31/55
//C07D487/06, C07D223/22

(30) Priority: 07.03.84 GB 8405954

(43) Date of publication of application: 11.09.85 Bulletin 85/37

(84) Designated Contracting States: BE CH DE FR GB IT LI NL SE

(71) Applicant: BEECHAM GROUP PLC
Beecham House Great West Road
Brentford Middlesex TW8 9BD(GB)

(72) Inventor: Newsome, Peter Martin
The Limes 20A York Road
Cheam Surrey(GB)

(72) Inventor: Beeley, Lee James
40 St. Johns Road Westcott
Dorking Surrey(GB)

(72) Inventor: Moss, Stephen Frederick
Blakehall Road
Carshalton Surrey(GB)

(72) Inventor: Bywater, Robert James
1 Shelvers Way
Tadworth Surrey(GB)

(74) Representative: Hardman, Carol Pauline et al,
Beecham Pharmaceuticals Great Burgh Yew Tree Bottom Road
Epsom Surrey KT18 5XQ(GB)

## (54) Treatment of diarrhoea.

(57) A method for treatment or preventing diarrhoea or scours which comprises administering to a human or non-human animal in need thereof an effective non toxic amount of a compound of formula (I);

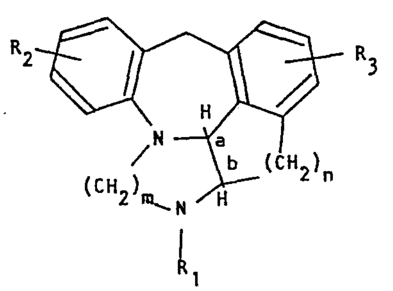

(I)

or an N-oxide or pharmaceutically acceptable quaternised derivative or pharmaceutically acceptable salt thereof wherein $R_1$ is hydrogen, $C_{1-7}$ alkyl, $C_{3-7}$ cycloalkyl, $C_{4-7}$ cycloalkenyl or $C_{1-4}$ alkyl substituted by $C_{2-7}$ alkenyl, $C_{2-7}$ alkynyl, $C_{3-7}$ cycoalkyl, hydroxy, thiol, $C_{1-4}$ alkoxy, $C_{1-4}$ alkylthio, carboxy, $C_{1-4}$ alkoxycarbonyl, $C_{1-4}$ alkanoyl, amino optionally substituted by one or two $C_{1-4}$ alkyl or by $C_{4-6}$ polymethylene optionally containing an oxygen or nitrogen atom, aminocarbonyl optionally N-substituted by one or two $C_{1-4}$ alkyl, or benzoyl or phenyl either being optionally ring-substituted by $C_{1-4}$ alkyl, $C_{1-4}$ alkoxy, halogen or trifluoromethyl, $R_2$ and $R_3$ are the same or different and are hydrogen, hydroxy, $C_{1-4}$ alkyl, $C_{1-4}$ alkoxy, $C_{1-4}$ alkylthio, halogen or trifluoromethyl, m is 1 to 3 and n is 1 or 2, the hydrogen atom bonded to the $C_a$ carbon atom being trans to the hydrogen atom bonded to the $C_b$ carbon atom.

Compounds and compositions for use in the method and for use in the preparation of medicaments are described.

EP 0 154 298 A2

# TREATMENT OF DIARRHOEA

The present invention relates to the treatment of diarrhoea using a class of pentacyclic compounds and to pharmaceutical formulations for use in such treatment.

European Published Patent Application No. 0088575 describes compounds of formula (I) below and N-oxides and pharmaceutically acceptable salts thereof, together with their use in the treatment of CNS disorders such as depression and anxiety. It has now surprisingly been found that compounds of formula (I) and certain derivatives thereof are also useful in protecting young animals from death due to neonatal scours and in the treatment of scours and diarrhoea.

Accordingly the present invention provides a method for treatment or preventing diarrhoea or scours which comprises administering to a human or non-human animal in need thereof an effective, non-toxic amount of a compound of formula (I):

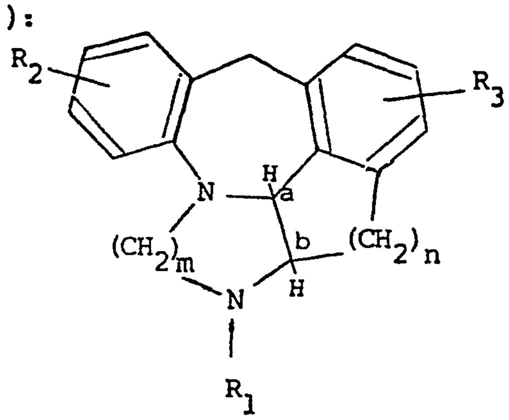

(I)

or an N-oxide or pharmaceutically acceptable quaternised derivative or pharmaceutically acceptable salt thereof, wherein $R_1$ is hydrogen, $C_{1-7}$ alkyl, $C_{3-7}$

cycloalkyl, $C_{4-7}$ cycloalkenyl or $C_{1-4}$ alkyl substituted by $C_{2-7}$ alkenyl, $C_{2-7}$ alkynyl, $C_{3-7}$ cycoalkyl, hydroxy, thiol, $C_{1-4}$ alkoxy, $C_{1-4}$ alkylthio, carboxy, $C_{1-4}$ alkoxycarbonyl, $C_{1-4}$ alkanoyl, amino optionally substituted by one or two $C_{1-4}$ alkyl or by $C_{4-6}$ polymethylene optionally containing an oxygen or nitrogen atom, aminocarbonyl optionally N-substituted by one or two $C_{1-4}$ alkyl, or benzoyl or phenyl either being optionally ring-substituted by $C_{1-4}$ alkyl, $C_{1-4}$ alkoxy, halogen or trifluoromethyl, $R_2$ and $R_3$ are the same or different and are hydrogen, hydroxy, $C_{1-4}$ alkyl, $C_{1-4}$ alkoxy, $C_{1-4}$ alkylthio, halogen or trifluoromethyl, m is 1 to 3 and n is 1 or 2, the hydrogen atom bonded to the $C_a$ carbon atom being trans to the hydrogen atom bonded to the $C_b$ carbon atom.

Within the definition for $R_1$ is a sub-group, wherein $R_1$ is hydrogen, $C_{1-4}$ alkyl, $C_{1-4}$ alkyl substituted by amino optionally substituted by one or two $C_{1-4}$ alkyl or by $C_{4-6}$ polymethylene optionally containing an oxygen or nitrogen atom, or $C_{1-4}$ alkyl substituted by phenyl optionally substituted by $C_{1-4}$ alkyl, $C_{1-4}$ alkoxy, halogen or trifluoromethyl.

When $R_1$ is $C_{1-4}$ alkyl substituted by phenyl optionally substituted as hereinbefore defined, examples of such optional substituents include methyl, ethyl, methoxy, ethoxy, fluoro, chloro, bromo or trifluoromethyl. Preferably, phenyl is unsubstituted.

When $R_1$ is $C_{1-4}$ alkyl substituted by amino optionally substituted as hereinbefore defined, examples of such optional substituents include methyl and ethyl and, together with the nitrogen atom, piperidino and morpholino.

Preferably, $R_1$ is hydrogen or $C_{1-4}$ alkyl, in particular $C_{1-4}$ alkyl, such as methyl and ethyl.

Within the definition for $R_2$ and $R_3$ is a sub-group, wherein $R_2$ and $R_3$ are the same or different and are hydrogen, $C_{1-4}$ alkyl, $C_{1-4}$ alkoxy, halogen or trifluoromethyl.

Preferred examples for $R_2$ and $R_3$ are hydrogen, hydroxy, methyl, ethyl, methoxy, ethoxy, bromo, chloro, fluoro and trifluoromethyl. Preferably, $R_2$ is hydrogen, methoxy, hydroxy, methyl or chloro and $R_3$ is hydrogen.

Preferably, m is 1 or 2.

Preferably, n is 1.

The compounds of the invention have chiral centres at the $C_a$ and $C_b$ carbon atoms and therefore can exist in enantiomeric forms. The present invention extends to such enantiomers individually and as mixtures including racemates.

Particularly preferred compounds within formula (I) are the compounds of the examples described hereinafter, or an N-oxide or pharmaceutically acceptable quaternised derivative or salt thereof. The most preferred compound of formula (I) is trans-12-methyl-1,10,11,12,12a,12b-hexahydro-5H-9b,12-diazabenzo[5,6]cyclohepta[1,2,3,4-def]fluorene, which is the compound prepared in Example 1, or a pharmaceutically acceptable quaternised derivative or pharmaceutically acceptable salt thereof.

Quaternised derivatives of compounds of formula (I) are novel and as such forms part of the invention.

Examples of pharmaceutically acceptable quaternised derivatives are the quaternary ammonium salts in particular those compounds quaternised by compounds such as $R_4$-Q wherein $R_4$ is optionally substituted $C_{1-6}$ alkyl, phenyl-$C_{1-6}$ alkyl or $C_{5-7}$ cycloalkyl, and Q is a radical corresponding to a anion of an acid. Suitable examples of $R_8$ include methyl, ethyl and _n_- and _iso_-propyl; and benzyl and phenethyl. Suitable Q include halide such as chloride, bromide and iodide.

Suitable substitutents for $R^4$ include hydroxy.

A preferred group of compounds of the present invention are compounds of formula (II)

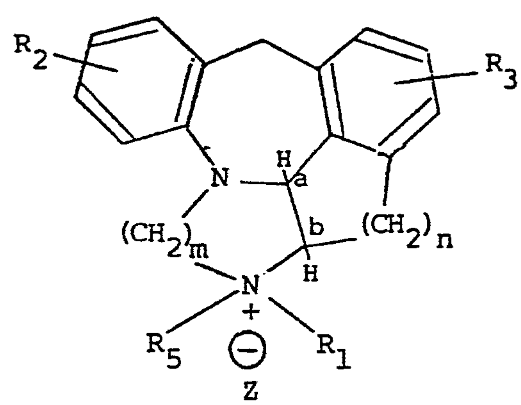

(II)

wherein $R_1$, $R_2$ and $R_3$ are as defined in relation to compounds of formula (I)

$R_5$ is $C_{1-6}$ alkyl which may optionally be substituted and $Z^-$ is a veterinarily or pharmaceutically acceptable anion.

Preferred groups $R_4$ include methyl, ethyl and hydroxyethyl.

Suitable veterinarily or pharmaceutically acceptable anions include halide ions of which iodide is preferred.

Preferred compounds of formula (II) include these wherein one or both $R^2$ and $R^3$ are hydrogen.

More preferred compounds of formula (II) are those wherein $R^1$ is methyl and m is 2.

An N-oxide of a compound of formula (I) includes the oxide of either nitrogen atom shown in formula (I) and the oxide of any nitrogen-containing substituent for $R_1$.

A pharmaceutically acceptable salt of a compound of formula (I) includes an acid addition salt of either nitrogen atom shown in formula (I) and of any nitrogen-containing substituent for $R_1$, the acid addition salt being derived from a pharmaceutically acceptable inorganic or organic acid, such as hydrochloric acid, hydrobromic acid, sulphuric acid, maleic acid, citric acid and acetic acid. A pharmaceutically acceptable salt of a compound of formula (I) also includes alkali metal or alkaline earth metal salts of any carboxy-containing substituent for $R_1$. Examples of such salts include potassium, sodium, calcium and magnesium salts.

Compounds of formula (I) and N-oxides and pharmaceutically acceptable quaternised derivatives and salts thereof protect animals from the lethal consequences of enterotoxigenic E.coli infections and

are therefore useful in the treatment of hypersecretory diarrhoea and scours.

As used herein 'pharmaceutically acceptable' means suitable for administration to human or non-human animals.

The present invention further provides a pharmaceutical composition for use in the treatment or prevention of diarrhoea or scours, which composition comprises a compound of formula (I) or an N-oxide or pharmaceutically acceptable quaternised derivative or salt thereof (hereinafter referred to as the 'drug') and a pharmaceutically acceptable carrier therefor.

The present invention further provides a pharmaceutical composition comprising a quaternised derivative of a compound of formula (I) as hereinbefore defined and a pharmaceutically acceptable carrier therefor.

Yet further according to the present invention there is provided the use of a compound of formula (I) as hereinbefore defined in the manufacture of a medicament for the treatment or prevention of diarrhoea or scours.

Pharmaceutical compositions of the drug will, of course, be adapted for administration to the humans or animals to be treated. Thus, for example, the composition may be a shaped composition, such as a bolus, tablet or capsule. In such cases the pharmaceutically acceptable carrier will be chosen from the usual range of lubricants, dispersants, binders, fillers and the like. When these shaped compositions are for administration to cattle and pigs often they will weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of the drug in a suitable vehicle for use with an oral doser (this is a well known item of farm equipment, basically comprising a liquid reservoir, a mouthpiece adapted for insertion into animals mouths, and a pump mechanism whereby unit doses can be ejected form the reservoir through the mouthpiece). Conveniently the drug may be administered from an oral doser as an aqueous solution. Alternatively, the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I). It will be convenient to formulate these animal feed and drinking water compositions with a multi-dose of the drug so that the animal takes in an appropriate quantity of the drug along with its diet. It will also be convenient to present the composition of the invention as a pre-mix for addition to the feed or drinking water.

With human babies or young animals, a particularly useful technique is to blend their milk with the drugs of this invention.
The compositions of the invention may also be formulated for injection. In such cases the drug chosen is suitably dissolved in water for injection.

Often it will be appropriate to include in the compositions a further medicine such as an antibacterial agent for example an antibiotic such as a β-lactam antibiotic, for example amoxycillin or ampicillin, or neomycin or a sulphonamide such as sulfadoxin, an agent to alter intestinal motility such as loperamide or an agent to modify stool consistency such as pectin.

Treatment of diarrhoea and scours using the drug may be supplemented by oral rehydration therapy such as those described in U.K. Patent No. 1,581,826 and German Offenlegungsschrift No. 28 54 281, UK Patent Application No. 2 012 163A, US Patent No. 3 898 328, Nalin, D.R. and Cash, R.A., Bull. World Health Org., 43, 361 (1970), French Patent No. 2 467 599, UK Patent No. 1 465 308 and as described in 'Secretory Diarrhoea', Ed M. Field, J.S. Fordtran and S,G, Schultz, American Physiological Society, Maryland, 1980 pp 179-185 and Lancet, (1975) pp 79 and 80.
Conveniently the drug may be administered with the oral rehydration formulation. Alternatively it may be provided separately and administered simultaneously or sequentially with the oral rehydration formulation.

The amount of drug administered must, of course, be sufficient to bring about the desired effect and will also depend on the body weight of the recipient and the chosen route of administration. Suitably the drug is

administered at from 10μg/kg to 100mg/kg, preferably 10μg/kg to 10mg/kg. Thus, by way of example, useful dosage units of the composition for treating diarrhoea may contain 5 μg to 500 mg of the drug. Of course, it will be appreciated that many preferred compositions of the invention are in multi-dose form as, for the therapy of animals, it is often most desirable to be able rapidly to treat a number of animals. Such multi-dose compositions will contain, by way of example, at least 1 mg of the drug. Depending on the exact nature of the said multi-dose composition, often it will contain at least 50 mg of the drug, and on occasions as much as 1 g. Doses may be administered once or several times daily.

Compounds of formula (I) or an N-oxide or pharmaceutically acceptable salt thereof may be produced by any of the processes described in European Published Patent Application No. 0088575.

Suitably N-oxides are produced by treating the compound of formula (I) with an oxidising agent, such as hydrogen peroxide, in a suitable solvent, such as methanol, at elevated temperature.

Quaternised derivatives of compounds of formula (I) may be prepared by reaction of a compound of formula I with a conventional reagent for quaternising from a secondary or tertiary amine, such as the appropriate alkyl, aryl or aralkyl chloride, bromide or iodide. This reaction may be carried out in a solvent, such as acetone, methanol, ethanol, nitromethene, dimethylformamide, at ambient or elevated temperature with or without pressure.

For example a compound of formula (II), as hereinbefore defined is prepared by treating the appropriate compound of formula (I) with a compound of formula (III)

$$R_5\text{-}Z \qquad (III)$$

wherein $R_5$ and Z are as hereinbefore defined in suitable solvent such as nitromethane at elevated temperature and under an inert atmosphere.

Suitable compounds for use in the invention are selected from

trans-12-methyl-1,10,11,12,12a,12b-hexahydro-5H-9b,12-diazabenzo[5,6]cyclohepta[1,2,3,4-def]fluorene;

trans-13-methyl-1,2,6,11,12,13,13a,13b- octahydro-10b,13-diazabenzo[gh]pleidene;

trans-7-methoxy-12-methyl-1,10,11,12,12a,12b-hexahydro-5H-9b,12-diazabenzo[5,6]cycloahepta[1,2,3,4-def]fluorene;

trans-7,12-dimethyl-1,10,11,12,12a,12b-hexahydro-5H-9b,12-diazabenzo[5,6]cyclohepta[1,2,3,4-def]fluorene;

trans-8-chloro-12-methyl-1,10,11,12,12a,12b-hexahydro-5H-9b,12-diazabenzo[5,6]cyclohepta[1,2,3,4-def]fluorene;

trans-7-hydroxy-12-methyl-1,10,11,12,12a,12b-hexahydro-5H-9b,12-diazabenzo[5,6]cyclohepta[1,2,3,4-def]fluorene;

trans-1,10,11,12,12a,12b-hexahydro-5H-9b,12-diazabenzo[5,6] cyclohepta[1,2,3,4-def]fluorene;

trans-12-benzyl-1,10,11,12,12a,12b-hexahydro-5H-9b,12-diazabenzo[5,6]cyclohepta[1,2,3,4-def]fluorene;

trans-12-(prop-2-enyl)-1,10,11,12,12a,12b-hexahydro-5H-9b,12-diazabenzo[5,6]cyclohepta[1,2,3,4-def]fluorene;

trans-12-(prop-2-ynyl)-1,10,11,12,12a,12b-hexahydro-5H-9b,12-diazabenzo[5,6]cyclohepta[1,2,3,4-def]fluorene; and trans-12-(2-hydroxyethyl)-1,10,11,12,12a,12b-hexahydro- 5H-9b,12-diazabenzo[5,6]cyclohepta-[1,2,3,4-def] fluorene;

trans-12-(2-methoxyethyl)-1,10,11,12,12a,12b-hexahydro-5H-9b,12b-diazabenzo[5,6]cyclohepta[1,2,3,4-def]fluorene;

trans-12-ethyl-1,10,11,12,12a,12b-hexahydro-5H-9b,12-diazabenzo[5,6]cyclohepta[1,2,3,4-def]fluorene;

trans-12-cyclohexylmethyl-1,10,11,12,12a,12b-hexahydro-5H-9b,12-diazabenzo[5,6]cyclohepta[1,2,3,4-def] fluorene;

trans-12-(3-oxobutyl)-1,10,11,12,12a,12b-hexahydro-5H-9b,12-diazabenzo[5,6]cyclohepta[1,2,3,4-def]fluorene;

trans-12-(2-ethoxycarbonylethyl)-1,10,11,12,12a, 12b-hexahydro-5H-9b,12-diazabenzo[5,6]cyclohepta [1,2,3,4-def]fluorene;

trans-12-(2-dimethylaminoethyl)-1,10,11,12,12a,12b-hexahydro-5H-9b,12-diazabenzo[5,6]cyclohepta [1,2,3,4-def]fluorene;

or an N-oxide or pharmaceutically acceptable quaternised derivative or pharmaceutically acceptable salt thereof.

or an N-oxide or a pharmaceutically acceptable quarternised derivative or pharmaceutically acceptable salt thereof;

Suitable quaternised salts of the invention include:

1, 2, 3, 4, 10, 14b, hexahydro-2,2-dimethyl-1,14-methanodibenz [c,g] pyrazinium [1,2-a] azepine halide;

1, 2, 3, 4, 10, 14b-Hexahydro-2,2-dimethyl-1, 14-methanodibenzo [c,f]pyrazinium [1,2-a]azepine halide; and

1, 2, 3, 4, 10, 14b-hexahydro-2-(2-hydroxymethyl)-2-methyldibenzo [c,f] pyrazinium [1,2-a]azelpine halide.

The invention will now be illustrated by the following Examples:

All temperatures are in degrees celsius and 'Rec' means recrystallised from.

Description 1

trans-1-(2-Methoxycarbonylanilino)-2-ethoxycarbonylaminoindane (D4)

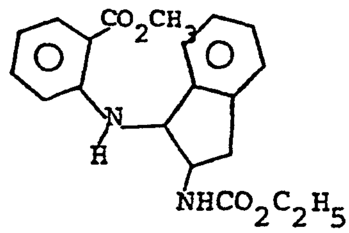

(D4)

trans-1-Chloro-2-ethoxycarbonylaminoindane (20g, 0.084 moles) was treated with methyl anthranilate (60 ml) and stirred under nitrogen at 60° for 5hr. The resulting viscous mixture was diluted with ether (500ml), washed exhaustively with 2.5N HCl (8x250ml), and then with saturated sodium bicarbonate followed by brine. After drying ($Na_2SO_4$) and concentration *in vacuo* a brown solid (25.7g) was obtained. Crystallisation from pentane/ether afforded the title compound (14g; 58%) m.p. 108-110°. Concentration of mother liquors gave a less pure second crop (2.6g).

Nmr ($CDCl_3$) δ: 1.21 (3H,t,J=7), 2.30 (1H,dd,J=16,6) 3.42 (1H,dd,J=16,7), 3.80 (3H,s), 3.9-4.5 (3H, m, overlapping signals), 4.92 (1H,d, J=5), 4.95 (2H, m, overlapping signals), 6.65 (1H, m), 7.26 (6H, m), 7.94 (1H,dd, J=9, 1.5).

Description 2

trans-1-(2-Hydroxymethylanilino)-2-ethoxycarbonyl-aminoindane (D5)

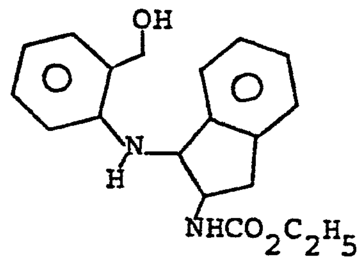

(D5)

A solution of the ester D4 (1.0g, 2.8 mmoles) in dry tetrahydrofuran (6ml) was cooled below $-10^{o}$ under nitrogen and treated dropwise with Super-hydride (Lithium triethylborohydride) (10 ml of a 1M Tetrahydrofuran solution). Stirring was continued overnight at room temperature. The reaction mixture was then cooled below $0^{o}$ and treated with water (1ml) followed by 5N HCl (25ml). After stirring for 30 mins. the mixture was diluted with pentane. The aqueous layer was washed with ether (2 x 20ml) basified (40% NaOH) and extracted into ether. The organic phase was washed (brine), dried ($Na_2SO_4$) and concentrated <u>in vacuo</u> to give the title compound as a colourless solid (0.75g, 81%) m.p. $115-7^{o}$ (rec. ether/pentane).

Nmr ($CDCl_3$) δ: 1.17 (3H,t,J=7), 2.76 (1H,dd,J=16,8), 3.34 (1H,dd,J=16,8), 4.02 (2H,q, J=7), 4.35 (1H,m), 4.62 (2H,s), 4.82 (1H,d,J=7), 4.95 (1H,broad), 6.5-7.5 (8H,m).

Description 3

trans-1-Ethoxycarbonylamino-1,2,11,11a-tetrahydro-6H-benzo[f]indeno[1,7-bc]azepine (D7)

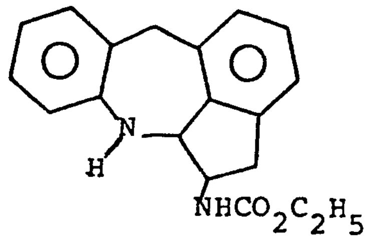

(D7)

The alcohol prepared in Description 2 (8.6g; 0.026 moles) was dissolved in methanesulphonic acid (86g; 58ml) and the cooled solution was treated with phosphorus pentoxide (17.2g) and stirred at room temperature for 4 days. The mixture was poured onto ice, neutralised to pH7 (40% NaOH) and extracted into ether. The organic layers were washed (water), dried ($Na_2SO_4$) and concentrated *in vacuo* to give a yellow foam (6.4g) containing two faster running products on tlc (Rf values 0.77 and 0.6 - $SiO_2$/petroleum ether/ether-3/1). The mixture was separated on silica gel using 20% ethyl acetate in petroleum ether as eluant. The more polar component corresponded to the title compound and was isolated as a colourless crystalline solid (1.87g; 23%).

Nmr ($CDCl_3$) δ: 1.28 (3H,t,J=7), 2,57 (1H,dd,J=16,10), 3.15 (1H,dd,J=16,8), 3.63 (1H,d,J=15), 4,25 (5H, overlapping signals), 4.68 (1H, d,J=8), 5.07 (1H, broad doublet), 6.5-7.3 (7H,m).

Description 4

trans-1-Ethoxycarbonylamino-11-bromoacetyl-1,2,11,11a-tetrahydro-6H-benzo[f]indeno[1,7-bc]azepine (D9)

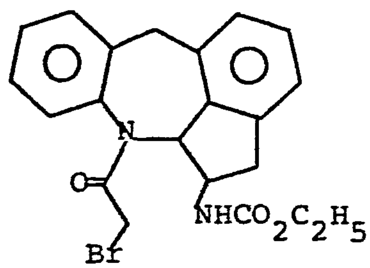

(D9)

Bromoacetyl bromide (0.88 ml; 0.01 moles) was added dropwise to a solution of the amine prepared in Description 3 (3.08g; 0.01 moles) in dry methylene chloride (25 ml) containing finely ground potassium carbonate (2.76g; 0.02 moles) and cooled to $0^{o}$. Stirring was continued for 27 hours and during this period a further portion (0.2 ml) of bromoacetyl bromide was added. The mixture was then treated with water and after separation of the organic phase the aqueous layer was extracted with methylene chloride. The combined organic layers were washed (water), dried, ($Na_2SO_4$) and concentrated in vacuo to give a yellow solid. Purification by trituration with pentane/ether afforded the title compound as a colourless crystalline solid (4.1g; 95%) m.p. 207.5-210°C (rec. ether).

Nmr ($CDCl_3$) δ: 1.33 (3H,t,J=7), 2.75 (1H,dd,H=15,10), 3.32 (1H,dd,J=16,8), 3.44 (1H,d,J=13), 3.80 (2H,s), 4.2 (4H,overlapping signals), 6.1 (2H,overlapping doublets) 6.8-7.5 (7H, m).

Description 5

trans-10-Oxo-1,10,11,12,12a,12b-Hexahydro-5H-9b,12-diaza-benzo[5,6]cyclohepta[1,2,3,4-def]fluorene-12-carboxylic acid ethyl ester (D11)

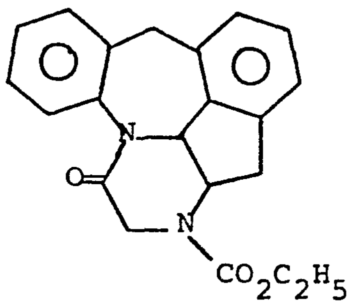

(D11)

A solution of the urethane prepared in Description 4 (3,35g; 7.8 mmoles) in dry dimethyl formamide (200ml) was added over a period of 30 minutes to a stirred suspension of sodium hydride (0.26g of 80% dispersion in oil; 8.6 mmoles) in the same dry solvent (20 ml) under nitrogen. Reaction temperature was maintained below 5$^{o}$ during addition and then allowed to rise to room temperature while stirring was continued for a further 3h. The mixture was then carefully diluted with water and extracted into ether. The organic phase was washed exhaustively with water, dried ($Na_2SO_4$) and concentrated to give the title compound as a pale yellow solid (2.35g; 90%) which was used without further purification.

Nmr ($CDCl_3$) δ: 1.35 (3H,t,J=7), 2.75 (1H,dd,J=16,10), 3,50 (1H,d,J=14), 3.55 (1H,dd,overlapping), 3.65-4.50 (4H,overlapping signals), 4.86 (1H,d,J=16), 5.35 (1H,d,J=11), 6.80-7.40 (6H,m), 7.60 (1H,m).

Description 5

trans-1,10,11,12,12a,12b-Hexahydro-5H-9b,12-diaza-benzo[5,6]cyclohepta[1,2,3,4-def]fluorene-12-carboxylic acid ethyl ester (D13)

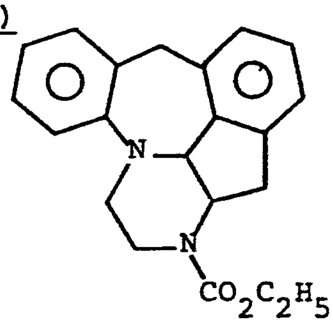

(D13)

A solution of the urethane prepared in Description 4 (2.2g; 6.3 mmoles) in dry tetrahydrofuran (15ml) was added dropwise to 10.5 ml of 1M diborane in tetrahydrofuran cooled to ice temperature under nitrogen. The solution was then refluxed for 2 hours. After cooling to $-10^{o}$ the mixture was carefully acidified (5N HCl) and stirred for 30 mins. Solvent was removed in vacuo and the residue treated with 2N NaOH before extraction into ether. The dried ($Na_2SO_4$) organic phase was concentrated in vacuo to give a foam (1.9g). Purification on silica gel using 15% ethyl acetate in petroleum ether 60/80 as eluant afforded the title compound as a colourless foam (1.45g; 70%).

Nmr ($CDCl_3$) δ: 1.32 (3H,t,J=7), 3.0-4.7 (12H,m), 6.6-7.3 (7H,m).

Example Ia

trans-12-Methyl-1,10,11,12,12a,12b-hexahydro-5H-9b,12-diazabenzo[5,6]cyclohepta[1,2,3,4-def]fluorene (E1)

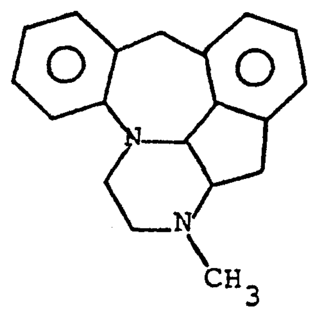

(E1)

A solution of the urethane prepared in Description 5 (1,4g; 4,0 mmoles) in dry tetrahydrofuran (10 ml) was added dropwise to a stirred suspension of lithium aluminium hydride (0.45g; 12.0 mmoles) in the same dry solvent (4ml) under nitrogen, and the mixture was refluxed for 50 mins. Excess hydride was destroyed with wet ether and after careful treatment with water the precipitate of aluminium oxides was filtered off and the filtrate concentrated in vacuo to give the title compound as a light yellow foam (0.98g; 89%) which crystallised on addition of acetone.m.p. 151-2$^{o}$ (from pentane/ethyl acetate).

Nmr δ: 2.20 (1H,ddd,J=10,10,6), 2.35 (3H,s), 2.50 (1H,ddd, J=12,12,3), 2.61 (1H,dd,J=14,11), 2.87 (1H,m), 2.88 (1H,dd,J=14,6), 3.45 (1H,d,J=13), 3.69 (1H,ddd,J= 14.5,11,3), 3.87 (1H,ddd,J=14,3,3), 4.37 (1H,d,J=13), 4.48 (1H,d,J=10), 6.7-7.3 (7H,m).

Treatment of the free base with 1 equivalent of maleic acid in acetone solution afforded the maleate salt. m.p. 183-5$^{o}$ (from acetone/ether).

| | C | H | N |
|---|---|---|---|
| Found | 70.32 | 6.25 | 7.01 |
| $C_{23}H_{24}N_2O_4$ Requires | 70.39 | 6.16 | 7.14 |

Example Ib(Alternative Procedure)

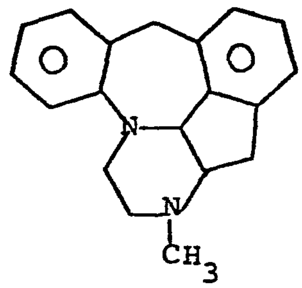

(EI).

trans-4-(2-Hydroxymethylphenyl)-1-methyl-2,3,4,4a,9,9a-hexahydro-1H-indeno[1,2-b]pyrazine (680g;2.31 moles) was added to stirring orthophosphoric acid (6.8 l of an 88% solution) at ca 90°. After 1h the mixture was poured onto a mixture of ice (20 kg) and chloroform (12.5 l) and stirred vigorously as 40% sodium hydroxide solution was carefully added to neutralise the acid while the temperature was maintained below 45°. The organic layer was separated and the aqueous phase extracted with two further portions of chloroform. The combined chloroform layers were washed (water), dried ($MgSO_4$) and concentrated in vacuo. Purification by flash chromatography, using pet. ether/acetone (70/30) as eluant, followed by crystallisation afforded the title compound as a colourless solid (560g; 88%). m.p. 151-2° (Rec twice pentane/ethyl acetate).

| | C | H | N |
|---|---|---|---|
| Found | 82.43 | 7.27 | 10.30 |
| $C_{19}H_{20}N_2$ Requires | 82.57 | 7.29 | 10.13 |

Example 2

1,2,3,4,10,14b-Hexahydro-2-methyl-1,14-methanodibenzo [c,f]pyrazino[1,2-a]azepine-2-oxide

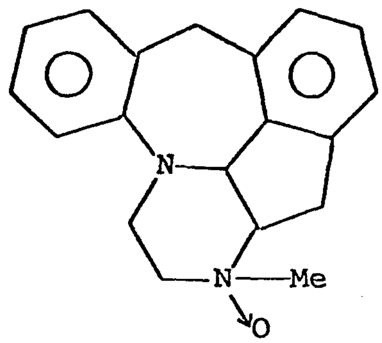

Three equal portions of 30% hydrogen peroxide solution (total volume taken 3.0 $cm^3$) were added at the start, after 3h and 6h to a stirred, refluxing solution of 1,2,3,4,10,14b-Hexahydro-2-methyl-1,14-methanodibenzo [c,f]pyrazino[1,2-a]azepine (3.6mmol) in methanol (50 $cm^3$). After 48h at reflux, the reaction mixture was cooled, stirred with a catalytic quantity of 5% platinum on carbon to destroy excess hyrogen peroxide, filtered and evaporated under vacuum. The crude material thus obtained was purified either by recrystallisation or column chromatography on silica gel. Thus the title dibenzopyrazinoazepine-N-oxide was obtained as white needles, m.pt. 203-4°C (dec.), in 81% yield by recrystallisation from 2-propanol-methanol-water.

Analysis calculated for $C_{19}H_{20}N_2O$

Theory: C, 78.05; H, 6.90; N, 9.58%

Found : C, 77.92; H, 6.82; N, 9.50%

Example 3

1,2,3,4,10,14b-Hexahydro-2,2-dimethyl-1,14-methanodibenzo[c,f]pyrazinium[1,2-a]azepine iodide

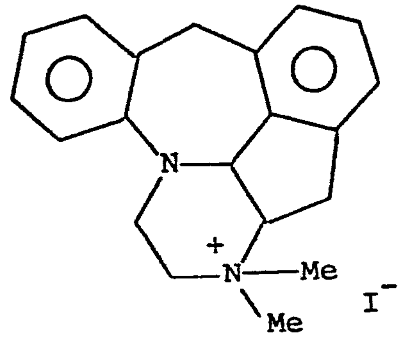

A mixture of 1,2,3,4,10,14b-Hexahydro-2-methyl-1, 14-methanodibenzo [c,f]pyrazino[1,2-a]azepine (1 mmol), iodomethane (3 mmol) and nitromethane (10 $cm^3$) was heated at 55°C for 2h then cooled to ambient temperature and stirred for 24h. Dilution of the reaction mixture with diethylether yielded the N,N-dimethyldibenzopyrazinium azepine iodide as a white precipitate which was purified by recrystallisation from methanol. Thus the title N,N-dimethyldibenzopyrazinium azepine iodide m.pt. 255-7°C (dec.) was obtained in 95% yield by crystallisation from methanol.

Analysis calculated for $C_{20}H_{23}I_2N_2$

Theory : C, 57.42; H, 5.54; N, 6.70%

Found : C, 57.26; H, 5.57; N, 6.66%

## Formulation of the Compounds for Veterinary Administration

As used in the following formulations 'drug' refers to the Compound of Example 1.

### Formulation 1: Bolus

Boluses of the following composition were prepared:-

| | |
|---|---|
| Drug | 10 mg |
| Microcrystalline cellulose | 500 mg |
| Corn starch | 250 mg |
| Magnesium stearate | 25 mg |
| Lactose, anhydrous | to 2500 mg |

The ingredients were passed through a 30 mesh stainless steel screen and blended in a suitable blender. The resultant compression mix was compressed directly on a tabletting machine to give tablets each containing 2 mg of the drug.

Formulation 2:

Oral Doser 1 mg/g

1 Kg of the following composition was prepared:-

| | % by wt. |
|---|---|
| Drug | 0.1 |
| Aluminium stearate | 6.0 |
| Sunflower oil | to 100 |

The aluminium stearate was dispersed with stirring in a portion of the sunflower oil heated to 115°C. The dispersion was added to the rest of the sunflower oil heated to 140°C. The gel was stirred at 130°C for 15 minutes and then allowed to cool without stirring to room temperature. The milled drug was dispersed in the cooled gel base and then passed through a colloid mill to produce a fine, homogenous dispersion. The dispersion was filled into plastic bottles fitted with a dosing pump.

Formulation 3

Injection 5 mg/ml

1 litre of the following composition was prepared:-

| | % w/v |
|---|---|
| Drug | 0.5 |
| Water for injections | to 100 |

The drug and sodium chloride were dissolved in the water for injections and the solution was filtered and filled into glass ampoules. The ampoules were sterilised by membrane filtration.

Formulation 4

Soluble Powder

1 Kg of the following composition was prepared:-

| | % by wt. |
|---|---|
| Drug | 0.7 |
| Lactose | to 100 |

The drug and lactose were sieved and mixed together in a suitable blender to give a homogenous powder. The powder was filled into jars. The powder was used at the rate 1 g per gallon of drinking water to medicate pigs.

Formulation 5

Oral Rehydration Formulation

1 kg of the following composition was prepared by mixing together the ingredients in dry powder form:

| | |
|---|---|
| Glycine | 10.3 % |
| Dextrose (anhydrous) | 67.6 |
| Sodium Chloride | 14.3 |
| Potassium Dihydrogen Phosphate | 6.8 |
| Citric Acid | 0.8 |
| Tri-potassium Citrate | 0.2 |
| Drug | 0.08 |

60 g of the composition was then mixed with 2 litres of warm water and fed to diarrhoeic calves.

Formulation 6

The following formulation may be prepared by the method set out below:-

| | |
|---|---|
| Drug | 0.1 % w/v |
| Bentone 38 (1) | 1.5 % w/v (ie 1.5 g/100ml) |
| Propylene Carbonate | 0.6 % w/v |
| Pharmasorb (2) | 10 % w/v |
| Phosphoric Acid (3) | 0.1 % w/v |
| Ampicillin Trihydrate | 6.0 % w/v as free acid |
| Soya-Bean Oil | to 100 % |

(1) Bentone 38 is an amide derivative of bentonite

(2) Pharmasorb is a brand of activated Attapulgite,

(3) The phosphoric acid is present to balance the alkaline pH of the Bentone.

The Bentone was dispersed in the soya-bean oil, and when thoroughly distributed, the propylene carbonate was added with high speed mixing, followed by colloid milling to produce the base. Into this base was first mixed the phosphoric acid, and then the pharmasorb, the penicillin, and the drug and the resultant suspension was then passed through a colloid mill once more.

## BIOLOGICAL EVALUATION

### (i) Protection of Neonatal Mice from Lethal Enteropathogenic E coli Infection

4 day old mice were orally dosed with 50µl of phosphate buffered saline containing 1 x $10^5$ organisms/ml of E coli B44 (09:K90:K99) an enteropathogenic strain originaly isolated from a scouring calf. The mice were then dosed b.i.d. with either placebo or drug for four days commencing 16 hours after infection. The animals were left with their mothers throughout the experiment and a daily record of deaths was made. The experiment was terminated 10 days after infection. The mortality in the drug treated group was then compared with the mortality in the placebo treated group using the following formula:-

$$\% \text{ Reduction in mortality} = \frac{Mp-Md}{Mp} \times 100$$

where Mp = % mortality in group receiving placebo
Md = % mortality in group receiving drug

Statistical analysis was performed using 2 x 2 contingency tables (single tailed 'p'). Results are given in Tables 1 and 2.

Table 1

| Compound of Example No. | Dose (mg/kg) | $n_p$ | $n_d$ | $M_p$ | $M_d$ | % reduction in mortality (significance) |
|---|---|---|---|---|---|---|
| 1 | 10 | 69 | 69 | 71 | 54 | 24 $(p<0.05)$ |
| | 2 | 70 | 73 | 61 | 36 | 42 $(p<0.01)$ |
| | 0.4 | 71 | 70 | 69 | 50 | 28 $(p<0.05)$ |

$n_p$ = no. infected mice in placebo treated group
$n_d$ = no. infected mice in drug treated group
$M_p$ = % mortality in placebo treated group
$M_d$ = % mortality in drug treated group

Table 2

| Compound of Example No. | Dose (mg/kg) | Reduction in mortality (%) | No of infected treated mice |
|---|---|---|---|
| 2 | 0.4 | 19 | 40 |
| 3 | 2.0 | 6 | 17 |

0154293

Eu, Dk

Claims

1. The use of a compound of formula (I),

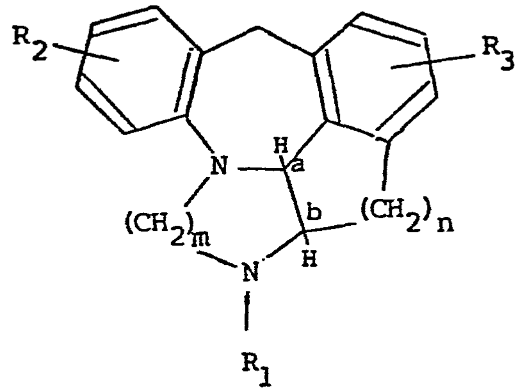

(I)

or an N-oxide or pharmaceutically acceptable quaternised derivative or pharmaceutically acceptable salt thereof, wherein $R_1$ is hydrogen, $C_{1-7}$ alkyl, $C_{3-7}$ cycloalkyl, $C_{4-7}$ cycloalkenyl or $C_{1-4}$ alkyl substituted by $C_{2-7}$ alkenyl, $C_{2-7}$ alkynyl, $C_{3-7}$ cycloalkyl, hydroxy, thiol, $C_{1-4}$ alkoxy, $C_{1-4}$ alkylthio, carboxy, $C_{1-4}$ alkoxycarbonyl, $C_{1-4}$ alkanoyl, amino optionally substituted by one or two $C_{1-4}$ alkyl or by $C_{4-6}$ polymethylene optionally containing an oxygen or nitrogen atom, aminocarbonyl optionally N-substituted by one or two $C_{1-4}$ alkyl, or benzoyl or phenyl either being optionally ring-substituted by $C_{1-4}$ alkyl, $C_{1-4}$ alkoxy, halogen or trifluoromethyl, $R_2$ and $R_3$ are the same or different and are hydrogen, hydroxy, $C_{1-4}$ alkyl, $C_{1-4}$ alkoxy, $C_{1-4}$ alkylthio, halogen or trifluoromethyl, m is 1 to 3 and n is 1 or 2, the hydrogen atom bonded to the $C_a$ carbon atom being trans

to the hydrogen atom bonded to the $C_b$ carbon atom; in the manufacture of a medicament for the treatment or prevention of diarrhoea or scours in human or non-human animals.

2. A use according to claim 1, wherein $R_1$ is hydrogen, $C_{1-4}$ alkyl, $C_{1-4}$ alkyl substituted by amino optionally substituted by one or two $C_{1-4}$ alkyl or by $C_{4-6}$ polymethylene optionally containing an oxygen or nitrogen atom, or $C_{1-4}$ alkyl substituted by phenyl optionally substituted by $C_{1-4}$ alkyl, $C_{1-4}$ alkoxy, halogen or trifluoromethyl.

3. A use according to claim 2, wherein $R_1$ is hydrogen or $C_{1-4}$ alkyl, such as methyl or ethyl.

4. A use according to any one of claims 1 to 3, wherein $R_2$ and $R_3$ are the same or different and are hydrogen, $C_{1-4}$ alkyl, $C_{1-4}$ alkoxy, halogen or trifluoromethyl.

5. A use according to any one of the preceding claims, wherein m is 1 or 2.

6. A use according to any one of the preceding claims, wherein n is 1.

7. A use according to claim 1 wherein the compound of formula (I) is

trans-13-methyl-1,2,6,11,12,13,13a,13b- octahydro-10b, 13-diazabenzo[gh]pleiadene;

trans-7-methoxy-12-methyl-1,10,11,12,12a,12b-hexahydro-5H-9b,12-diazabenzo[5,6]cycloahepta[1,2, 3,4-def]fluorene;

trans-7,12-dimethyl-1,10,11,12,12a,12b-hexahydro-5H-9b, 12-diazabenzo[5,6]cyclohepta[1,2,3,4-def]fluorene;

trans-8-chloro-12-methyl-1,10,11,12,12a,12b-hexahydro-5H-9b,12-diazabenzo[5,6]cyclohepta[1,2,3,4-def] fluorene;

trans-7-hydroxy-12-methyl-1,10,11,12,12a,12b-hexahydro-5H-9b,12-diazabenzo[5,6]cyclohepta[1,2,3,4-def] fluorene;

trans-1,10,11,12,12a,12b-hexahydro-5H-9b,12-diazabenzo [5,6] cyclohepta[1,2,3,4-def]fluorene;

trans-12-benzyl-1,10,11,12,12a,12b-hexahydro-5H-9b, 12-diazabenzo[5,6]cyclohepta[1,2,3,4-def]fluorene;

trans-12-(prop-2-enyl)-1,10,11,12,12a,12b-hexahydro-5H-9b,12-diazabenzo[5,6]cyclohepta[1,2,3,4-def]fluorene;

trans-12-(prop-2-ynyl)-1,10,11,12,12a,12b-hexahydro-5H-9b,12-diazabenzo[5,6]cyclohepta[1,2,3,4-def]fluorene;

trans-12-(2-hydroxyethyl)-1,10,11,12,12a,12b-hexahydro-5H-9b,12-diazabenzo[5,6]cyclohepta[1,2,3,4-def] fluorene;

trans-12-(2-methoxyethyl)-1,10,11,12,12a,12b-hexahydro-5H-9b,12b-diazabenzo[5,6]cyclohepta[1,2,3, 4-def]fluorene;

trans-12-ethyl-1,10,11,12,12a,12b-hexahydro-5H-9b,12-diazabenzo[5,6]cyclohepta[1,2,3,4-def]fluorene;

trans-12-cyclohexylmethyl-1,10,11,12,12a,12b-hexahydro-5H-9b,12-diazabenzo[5,6]cyclohepta[1,2,3,4-def] fluorene;

trans-12-(3-oxobutyl)-1,10,11,12,12a,12b-hexahydro-5H-9b,12-diazabenzo[5,6]cyclohepta[1,2,3,4-def]fluorene;

trans-12-(2-ethoxycarbonylethyl)-1,10,11,12,12a,12b-hexahydro-5H-9b,12-diazabenzo[5,6]cyclohepta[1,2,3,4-def]fluorene;
trans-12-(2-dimethylaminoethyl)-1,10,11,12,12a,12b-hexahydro-5H-9b,12-diazabenzo[5,6]cyclohepta[1,2,3,4-def]fluorene;
or an N-oxide or pharmaceutically acceptable quaternised derivative or pharmaceutically acceptable salt thereof.

8. A use according to claim 1 wherein the compound of formula (I) is trans-12-methyl-1,10,11,12,12a,12b-hexahydro-5H-9b,12-diazabenzo[5,6]cyclohepta[1,2,3,4-def]fluorene; or an N-oxide or pharmaceutically acceptable quaternised derivative or pharmaceutically acceptable salt thereof.

9. A pharmaceutically acceptable quaternised derivative of a compound of formula I as defined in claim 1.

10. A pharmaceutical composition, which comprises a pharmaceutically acceptable quaternised derivative of a compound of formula (I), as defined in claim 11, a pharmaceutically acceptable carrier.

11. A pharmaceutical composition which comprises a compound of formula (I) as defined in claim 1 and an oral rehydration agent or antibiotic, in combination with a pharmaceutically acceptable carrier.

12. A pharmaceutical composition according to claim 11 wherein the compound of formula (I) is of trans-12-methyl-1,10,11,12,12a,12b-hexahydro-5H-9b,12-diazabenzo[5,6]cyclohepta[1,2,3,4-def]fluorene.